# EUROPEAN PATENT SPECIFICATION

(11) **EP 3 880 160 B1**
(45) Date of publication and mention of the grant of the patent: **01.03.2023**
(21) Application number: 19828114.9
(22) Date of filing: 05.12.2019
(51) Int. Cl.: A61K 8/19, A61K 8/21, A61K 8/27, A61Q 11/00

(54) **DENTIFRICE CONTAINING SODIUM BICARBONATE AND STANNOUS IONS.**
ZAHNCREMEZUSAMMENSETZUNG, UMFASSEND NATRIUMBICARBONAT UND ZINN IONEN.
DENTIFRICE COMPRENANT DU BICARBONATE DE SODIUM ET DES IONS STANNEUX

(30) Priority: 20.12.2018 US 201862783028 P
(43) Date of publication of application: 22.09.2021
(73) Proprietor: Colgate-Palmolive Company, New York, NY 10022 (US)
(72) Inventor: EVANS, Lauren, Highland Park, New Jersey 08904 (US); PATEL, Neeta Atul, Monmouth Junction, New Jersey 08852 (US); PAPPAS, Iraklis, Pennsauken, New Jersey 08109 (US); HU, Zhichao, Piscataway, New Jersey 08854 (US); THOMSON, Paul, Piscatatway, New Jersey 08854 (US)
(74) Representative: Sonnenhauser, Thomas Martin
(86) International application number: PCT/US2019/064610
(87) International publication number: WO 2020/131401

(56) References cited:
- EP-A1- 2 374 446
- WO-A1-88/10110
- WO-A1-98/02135
- WO-A1-2015/095932
- US-A- 4 828 822
- US-A1- 2004 115 138

## Description

### BACKGROUND

Stannous fluoride is well known for use in clinical dentistry with a history of therapeutic benefits dating back to the early 1950s. Stannous fluoride has been reported to be an effective agent for treating various oral conditions and diseases including plaque, gingivitis, sensitivity, enamel decalcification, and periodontitis, among others. Because stannous ion (tin II) rapidly oxidizes to stannic ion (tin IV) which is no longer bioactive, maximizing the amount of tin in the stannous form (tin II) is essential to providing these oral health benefits over the shelf life of the product. Thus, stannous fluoride formulations typically include stabilization systems designed to maintain the stannous ion in the tin (II) form. One approach to stabilizing stannous fluoride is reducing the amount of water present in the composition. However, reducing the level of water, and optionally replacing some or all of the removed water with a humectant, may create problems in obtaining acceptable rheology and thickening properties in the composition. Another approach to stabilizing stannous fluoride is adding chelating agents (e.g., gluconate, polyphosphate and citrate) into the composition. Although several methods of stabilizing stannous ion have been known in the art, both maximization and stabilization of tin in the stannous form (tin II) in dentifrice formulations has remained an ongoing challenge.

Accordingly, there exists a need for stabilized stannous fluoride formulations with improved oral health benefits. US 2004/0115138 A1 discloses a dentifrice gel or paste comprising from about 40% to 70% by weight baking soda, from about 0.5% to 5% by weight of a blend of natural ingredients, from about 0.5% to 5% by weight of a flavoring oil and a sufficient amount of a stabilizing combination of a xanthan gum binder, at least one betaine surfactant and a humectant comprising glycerin or glycerin and sorbitol in an amount sufficient to render the dentifrice formulation stable to degradation and resistant to syneresis and phase separation. WO 2015/095932 A1 discloses improved complexes of amorphous calcium phosphate and/or amorphous calcium fluoride phosphate stabilised by phosphopeptides/phosphoproteins by addition of stannous ions. EP 2 374 446 A1 discloses an oral care composition comprising a) an aqueous phase; b) stannous ions solvated in the aqueous phase; c) nitrates solvated in the aqueous phase; wherein the total content of said nitrates is such that the molar amount of nitrogen in the aqueous phase, measurable as nitrate, is less than 2 times the molar amount of solvated stannous ions; and d) a flavour substance, which is preferably solvated, dispersed or emulgated in the aqueous phase. WO 98/02135 A1 discloses a stannous ion-containing composition for oral care containing a soluble stannous salt, an insoluble stannous salt and an acyl sarcosinate salt in an amount sufficient to effectively stabilize the stannous ion concentration. US 4,828,822 A discloses formulations for oral care and dental care, which contain tin (II) salts and water, are stabilized against hydrolysis of the tin salts by the addition of certain cationic surfactant diamine salts. WO 88/10110 A1 discloses a substantially water free tablet when chewed in the mouth forms a self-foaming paste containing stannous fluoride, which tablet contains less than about 50 % by weight of a composition producing carbon dioxide when mixed with the saliva in the mouth, and greater than about 35 % by weight of a substantially insoluble filling and polishing composition, and a wetting and foam stabilizing composition which forms the paste.

### BRIEF SUMMARY

The present disclosure provides dentifrice compositions, e.g., toothpaste or gel, comprising a stannous ion source (e.g., stannous fluoride) and sodium bicarbonate in an amount of greater than 50% by weight of the composition. In some embodiments, sodium bicarbonate is present in an amount of from 50% to 90%, from 60% to 80%, from 65% to 80%, from 65% to 75%, from 65% to 70%, or about 67% by weight of the composition. In some embodiments, the stannous ion source is present in an amount of from 0.01 % to 10%, e.g., from 0.1 % to 5 %, from 1 % to 5 %, from 1.5 to 4 %, from 0.1 % to 1 %, from 0.1 % to 0.2 %, from 0.2% to 0.8%, from 0.2 % to 0.5 %, or from 0.3 % to 0.6 %, or from 0.4% to 0.5% by weight of the composition. In a preferred embodiment, the stannous ion source is stannous fluoride.

In some embodiments, the dentifrice composition of the present invention further comprises zinc oxide. In some embodiments, zinc oxide is present in an amount of from 0.5 % to 2%, e.g., from 0.5% to 1.5%, from 0.8% to 1.3%, from 1% to 1.2%, from 1.1 % to 1.3%, about 1% or about 1.2% by weight of the composition.

A dentifrice composition as disclosed herein for use in a method to (i) reduce or inhibit formation of dental caries, (ii) reduce, repair or inhibit pre-carious lesions of the enamel, (iii) reduce or inhibit demineralization and promote remineralization of the teeth, (iv) reduce hypersensitivity of the teeth, (v) reduce or inhibit gingivitis, (vi) promote healing of sores or cuts in the oral cavity, (vii) reduce levels of acid producing bacteria, (viii) reduce or inhibit microbial biofilm formation in the oral cavity, (ix) reduce or inhibit plaque formation in the oral cavity, (x) promote systemic health, or (xi) clean teeth and oral cavity, is also part of the claimed invention.

Sodium bicarbonate in a dentifrice composition comprising a stannous ion source are used for increasing the stability of stannous ion in the composition, wherein sodium bicarbonate is present in an amount of greater than 50% by weight of the composition.

Further areas of applicability of the present disclosure will become apparent from the detailed description provided hereinafter.

### DETAILED DESCRIPTION

The following description of the preferred embodiment(s) is merely exemplary in nature and is in no way intended to limit the invention, which is defined in the claims, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present invention provides, in an aspect, a dentifrice composition, for example oral gel or toothpaste, that comprises a stannous ion source and sodium bicarbonate in an amount of greater than 50% by weight of the composition.

For example, the invention can include: sodium bicarbonate present in an amount of from 50% to 90%, from 60% to 80%, 65% to 80%, 65% to 75%, 65% to 70% or about 67% by weight of the composition.

The stannous ion source can be selected from the group consisting of stannous fluoride, stannous gluconate, stannous phosphate, stannous pyrophosphate, stannous acetate, stannous sulfate, stannous chloride and a combination thereof.

The stannous ion source can be stannous fluoride.

The composition can further comprise a stannous ion source which is not stannous fluoride.

The stannous ion source can be present in an amount of from 0.01 % to 10%, e.g., from 0.1 % to 5 %, from 1 % to 5 %, from 1.5 to 4 %, from 0.1 % to 1 %, from 0.1 % to 0.2 %, from 0.2% to 0.8%, from 0.2 % to 0.5 %, from 0.3 % to 0.6 %, or from 0.4% to 0.5% by weight of the composition.

The composition can further comprise a zinc ion source.

The zinc ion source can be selected from the group consisting of zinc oxide, zinc sulfate, zinc chloride, zinc citrate, zinc lactate, zinc gluconate, zinc malate, zinc tartrate, zinc carbonate, zinc phosphate and a combination thereof.

The zinc ion source can be present an amount of from 0.01 % to 5 %, e.g., 0.1% to 4%, or 0.5% to 3%, by weight of the composition.

The composition can comprise zinc oxide.

Zinc oxide can be present in an amount of from 0.5 % to 2%, e.g., from 0.5% to 1.5%, from 0.8% to 1.3%, from 1% to 1.2%, from 1.1 % to 1.3%, about 1%, or about 1.2% by weight of the composition.

The composition can comprise zinc oxide and zinc citrate.

Zinc oxide can be present in an amount of from 0.5 % to 2%, e.g., from 0.5% to 1.5%, from 0.8% to 1.3%, from 1% to 1.2%, from 1.1 % to 1.3%, about 1%, or about 1.2% by weight of the composition and zinc citrate is present in an amount of from 0.1% to 1%, from 0.25% to 0.75%, from 0.3% to 0.6%, about 0.5% by weight of the composition.

Zinc oxide can be present in an amount of about 1% by weight of the composition and zinc citrate is present in an amount of about 0.5% by weight of the composition.

The composition can comprise zinc phosphate.

Zinc phosphate can be present in an amount of from 0.5 % to 2%, e.g., from 0.5% to 1.5%, from 0.8% to 1.3%, from 1% to 1.2%, from 1.1 % to 1.3%, about 1%, or about 1.2% by weight of the composition.

The composition can comprise a fluoride ion source is selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and a combination thereof.

The fluoride ion source can be stannous fluoride.

The composition can further comprise a fluoride ion source which is not stannous fluoride.

The composition can comprise fluoride ion sources in amounts sufficient to supply 25 ppm to 5,000 ppm of fluoride ions, generally at least 500 ppm, e.g., 500 to 2000 ppm, e.g., 1000 ppm to 1600 ppm, e.g., 1450 ppm.

The composition can comprise one or more thickeners, for example thickening silicas.

The composition can comprise a foaming agent, for example a betaine, for example cocamidopropyl betaine.

The composition can comprise ingredients selected from one or more of buffering agents, humectants, surfactants, gum strips or fragments, breath fresheners, flavoring, fragrance, coloring, antibacterial agents, whitening agents, agents that interfere with or prevents bacterial attachment, calcium sources, and potassium salts.

The compositions can comprise water in an amount of from 1% to 20%, e.g., from 5% to 15%, 5% to 10%, from 7% to 15%, or from 11% to 13% by weight of the composition.

The composition can have a pH of from pH 7.5 to pH 10, preferably from pH 7.5 to pH 8.5, e.g., pH 7.6 to pH 8.4, pH 7.7 to pH 8.3, pH 7.8 to pH 8.1, or, e.g., about pH 8.0.

The composition can comprise stannous fluoride in an amount of from 0.4% to 0.5% by weight and zinc oxide in an amount of from 1% to 1.2% by weight of the composition.

The composition can be free of gluconate.

The composition can be free of polyphosphate.

The composition can be free of citrate.

The dentifrice composition can be free of any chelator (e.g., gluconate, polyphosphate and citrate) that chelates stannous ions.

The composition can be a toothpaste or gel.

The composition can be a toothpaste.

The composition can be a gel.

The compositions is for use in methods that (i) reduce or inhibit formation of dental caries, (ii) reduce, repair or inhibit pre-carious lesions of the enamel, (iii) reduce or inhibit demineralization and promote remineralization of the teeth, (iv) reduce hypersensitivity of the teeth, (v) reduce or inhibit gingivitis, (vi) promote healing of sores or cuts in the oral cavity, (vii) reduce levels of acid producing bacteria, (viii) reduce or inhibit microbial biofilm formation in the oral cavity, (ix) reduce or inhibit plaque formation in the oral cavity, (x) promote systemic health, or (xi) clean teeth and oral cavity.

Sodium bicarbonate can be used in a dentifrice composition comprising a stannous ion source for increasing the stability of stannous ion in the composition wherein sodium bicarbonate is present in an amount of greater than 50% by weight of the composition. Sodium bicarbonate can be present in an amount of from 50% to 90%, from 60% to 80%, 65% to 80%, 65% to 75%, 65% to 70% or about 67% by weight of the composition. In some embodiments, the composition comprises zinc oxide.

It has been surprisingly found that a high proportion of tin is present in the stannous form (tin II) when a high level of sodium bicarbonate (e.g., greater than 50%) is added to a dentifrice containing a stannous ion source. Without intending to be bound to theory, it is believed that the increased stability of stannous ion is due to the formation of insoluble stannous bicarbonate and/or stannous hydroxide due to the presence of sodium bicarbonate and the resulting high pH of the formulation. Indeed, it has been found that the soluble level of zinc and tin is low in the dentifrice composition containing a high level of sodium bicarbonate. It is believed that insoluble metal salts are inherently more resistant to oxidation due to slower kinetics in the solid-state form and are therefore better able to withstand oxidation during the dentifrice manufacturing process and during the aging of the formulation. It has been further found that the addition of zinc oxide into the dentifrice formulation containing a stannous ion source and a high amount of sodium bicarbonate improves the stability of stannous ion (tin II) during the aging of the formulation. Without intending to be bound to theory, it is believed that insoluble zinc oxide acts as a way of stabilizing stannous ion via adsorption of stannous ion on the surface of zinc oxide particle. The atomic sizes of zinc and tin are surprisingly similar (142 pm vs. 145 pm), considering the difference in atomic weights of the two metals. It is possible that stannous ions may have an acceptable size to enable them to be stabilized on the surface of the insoluble zinc oxide particle and prevented from oxidation.

In addition, it has been found that despite low solubility of zinc and tin in the dentifrice composition comprising a high level of sodium bicarbonate, the sodium bicarbonate dentifrice composition containing stannous fluoride and zinc oxide has a high biofilm inhibiting efficacy. Without intending to be bound to theory, it is believed that as there is no chelating agent in the formulation, the metals that gets dissolved are available in an unchelated form and therefore bioavailable to interact with bacteria. In addition, a high amount of sodium bicarbonate can also disrupt plaque biofilms. It is possible that the combination of sodium bicarbonate with stannous and zinc ions allows greater penetration of the metals into the biofilm, therefore enhancing their efficacy.

Thus, the present invention provides dentifrice compositions comprising a stannous ion source and sodium bicarbonate in an amount of greater than 50% by weight of the composition. The term "dentifrice" as used herein means paste or gel formulations unless otherwise specified. The dentifrice composition may be in any desired form, such as deep striped, surface striped, multi-layered, having the gel surrounding the paste, or any combination thereof. In some embodiments, the dentifrice composition is a toothpaste.

In some embodiments, sodium bicarbonate is present in an amount of from 50% to 90%, from 60% to 80%, 65% to 80%, 65% to 75%, 65% to 70% or about 67% by weight of the composition. Sodium bicarbonate (NaHCO₃), also known as baking soda, has been long used in dental care for the purpose of reducing plaque and whitening teeth, and further for reducing oral malodor. Sodium bicarbonate can buffer plaque acids, which cause demineralization of teeth, by returning the oral pH to a more favorable pH. In high concentrations, it is bactericidal against most periodontal pathogens. Sodium bicarbonate is also a desirable abrasive for dentifrice compositions because it is low in abrasion and imparts an exceptionally clean, fresh feel to the mouth. Sodium bicarbonate particles are relatively soft as compared to most conventional abrasive materials used in dentifrice compositions. In some embodiments, sodium bicarbonate is used as the sole abrasive in the composition. Such formulations remove plaque effectively, have a desirable low abrasivity, and provide an exceptionally clean feeling to the teeth and gums after brushing.

In some embodiments, the compositions of the present invention further comprise other conventional abrasives or polishing materials as a secondary abrasive. Conventional abrasives or polishing materials that may be useful herein as a secondary abrasive include e.g., silica abrasives, calcium phosphate abrasives, e.g., tricalcium phosphate (Ca₃(PO₄)₂), hydroxyapatite (Ca₁₀(PO₄)₆(OH)₂), or dicalcium phosphate dihydrate (CaHPO₄ · 2H₂O, also sometimes referred to as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

In some embodiments, the stannous ion source is selected from the group consisting of stannous fluoride, stannous gluconate, stannous phosphate, stannous pyrophosphate, stannous acetate, stannous sulfate, stannous chloride and a combination thereof. Stannous ion sources are well known in the art and may be incorporated into the compositions of the present invention. In some embodiments, stannous ion sources are present in the dentifrice composition in an amount of from 0.01 % to 10%, e.g., ., from 0.1 % to 5 %, from 1 % to 5 %, from 1.5 to 4 %, from 0.1 % to 1 %, from 0.1 % to 0.2 %, from 0.2% to 0.8%, from 0.2 % to 0.5 %, from 0.3 % to 0.6 %, or from 0.4% to 0.5% by weight of the composition. However, it is to be understood that the weights of stannous salts to provide the appropriate level of stannous ion will obviously vary based on the weight of the counter ion in the salt, and one of skill in the art may readily determine such amounts. In preferred embodiments, the stannous ion source is stannous fluoride. In some embodiments, stannous fluoride is present in an amount of in an amount of from 0.01 % to 10%, e.g., from 0.5 % to 7 %, from 1 % to 5 %, from 1.5 to 4 %, from 0.1 % to 1 %, from 0.1 % to 0.2 from 0.2% to 0.8%, from 0.2 % to 0.5 %, from 0.3 % to 0.6 %, or from 0.4% to 0.5% by weight of the composition. In some embodiments, the dentifrice composition may further contain other stannous ion source which is not stannous fluoride.

In some embodiments, the dentifrice compositions of the present invention comprise a fluoride ion source. Preferably, the fluoride ion source is stannous fluoride. In some embodiments, the dentifrice composition may further contain other fluoride which is not stannous fluoride. Representative fluoride ion sources include, but are not limited to, sodium fluoride, potassium fluoride, sodium monofluorophosphate, sodium fluorosilicate, ammonium fluorosilicate, amine fluoride, ammonium fluoride, and combinations thereof. In some embodiments, the composition may contain fluoride ion sources in amounts sufficient to supply 25 ppm to 5,000 ppm of fluoride ions, generally at least 500 ppm, e.g., 500 to 2000 ppm, e.g., 1000 ppm to 1600 ppm, e.g., 1450 ppm. Fluoride ion sources may be added to the compositions of the invention at a level of 0.01 % to 10 %, e.g., 0.03 % to 5 %, or 0.1 % to 1 %, by weight of the composition. However, it is to be understood that the weights of fluoride salts to provide the appropriate level of fluoride ion will obviously vary based on the weight of the counter ion in the salt, and one of skill in the art may readily determine such amounts.

The dentifrice compositions of the present invention may include one or more zinc ion sources. Zinc ions have been found to help in the reduction of gingivitis, plaque, sensitivity, and improved breath benefits. The zinc ion source can be a soluble or sparingly soluble compound of zinc with inorganic or organic counter ions. Examples include zinc oxide, zinc sulfate, zinc chloride, zinc citrate, zinc lactate, zinc gluconate, zinc malate, zinc tartrate, zinc carbonate, and zinc phosphate. In some embodiments, the zinc ion source is present in an amount of from 0.01 % to 5 %, e.g., 0.1% to 4%, or 1% to 3%, by weight of the composition.

In preferred embodiments, the dentifrice composition comprises zinc oxide. Zinc oxide may be present in an amount of 0.5 % to 2%, e.g., 0.5% to 1.5%, about 1% or about 1.2% by weight of the composition. In some embodiments, the composition comprises zinc oxide and zinc citrate. The compositions may comprise zinc oxide in an amount of 0.5 % to 2%, e.g., 0.5% to 1.5%, about 1% or about 1.2% by weight of the composition and zinc citrate in an amount of 0.1%-1%, 0.25-0.75%, about 0.5% by weight of the composition. In some embodiments, the compositions comprise zinc oxide in an amount of 1% by weight of the composition and zinc citrate in an amount of 0.5% by weight of the composition.

In some embodiments, the dentifrice composition comprises zinc phosphate. In some embodiments, the composition may comprise zinc phosphate in an amount of 0.5 % to 2%, e.g., 0.5% to 1.5%, about 1% or about 1.2% by weight of the composition.

The dentifrice compositions of the present invention may include other active ingredients. The active ingredients include, for example, anti-bacterial active agents, anti-tartar agents, anti-caries agents, anti-inflammatory agents, anti-sensitivity agents, basic amino acids, e.g., arginine, enzymes, nutrients, and the like. Actives useful herein are optionally present in the compositions of the present invention in safe and effective amounts that are sufficient to have the desired therapeutic or prophylactic effect in the human or lower animal subject to whom the active is administered, without undue adverse side effects (such as toxicity, irritation, or allergic response), commensurate with a reasonable risk/benefit ratio when used in the manner of this invention. The specific safe and effective amount of the active will vary with such factors as the particular condition being treated, the physical condition of the subject, the nature of concurrent therapy (if any), the specific active used, the specific dosage form, the carrier employed, and the desired dosage regimen.

In some embodiments, the dentifrice composition may comprise an aqueous buffer system for the source of stannous ions. The buffer system may be adapted to chelate the stannous ions in the composition. The buffer system may comprise at least one of a weak organic acid or an alkali metal salt thereof, the organic acid preferably being citric acid. The buffer system may comprise a mixture of citric acid and trisodium citrate. The buffer system may comprise from 0.1% to 10% by weight of the composition, e.g., 1 to 5 weight % of the composition. The buffer system may be present, by weight, in an amount that is greater than the amount, by weight, of the source of stannous ions. "Aqueous buffer system", as used herein, refers to the acidic and/or basic components of a buffer system that would result in an aqueous buffer system when the composition is dissolved or suspended in water.

The use of the buffer system described herein is believed to reduce or eliminate precipitation of insoluble tin compounds. An aqueous buffer system, e.g. a citrate buffer system, which may be employed as a premix for the stannous salt to chelate the stannous ions, can reduce or eliminate the precipitation of insoluble tin compounds in the presence of zinc ions and polyphosphates in a low water dentifrice composition.

A high level of sodium bicarbonate can stabilize stannous ions in a dentifrice composition without other stabilizing methods. Thus, the dentifrice composition, in some embodiments, does not contain any chelator (e.g., gluconate, polyphosphate and citrate) that chelates stannous ions.

The dentifrice compositions of the present invention may include one or more agents to increase the amount of foam that is produced when the oral cavity is brushed. Such foaming agents are known to those of skill in the art. Illustrative examples of agents that increase the amount of foam include, but are not limited to polyoxyethylene and certain polymers including, but not limited to, alginate polymers. The polyoxyethylene may increase the amount of foam and the thickness of the foam. Polyoxyethylene is also commonly known as polyethylene glycol ("PEG") or polyethylene oxide. The polyoxyethylenes suitable for this invention will have a molecular weight of 200,000 to 7,000,000, e.g., 600,000 to 2,000,000 or 800.000 to 1,000,000. The polyoxyethylene may be present in an amount of 1% to 90%, e.g., 5% to 50% or 10% to 20%, by weight of the composition. The dosage of foaming agent in the composition (i.e., a single dose) is 0.01 to 0.9 %, e.g., 0.05 to 0.5% or 0.1 to 0.2 %, by weight of the composition.

The dentifrice compositions of the present invention may include at least one surfactant or solubilizer. Suitable surfactants include neutral surfactants (such as polyoxyethylene hydrogenated castor oil or fatty acids of sugars), anionic surfactants (such as sodium lauryl sulfate), cationic surfactants (such as the ammonium cation surfactants) or zwitterionic surfactants. These surfactants or solubilizers may be present in amounts of typically 0.01% to 2%; or from 1% to 2%; or about 1.5%, by weight of the composition.

The dentifrice compositions of the present invention may include a sweetener such as, for example, saccharin, for example sodium saccharin, acesulfam, neotame, cyclamate or sucralose; natural high-intensity sweeteners such as thaumatin, stevioside or glycyrrhizin; or such as sorbitol, xylitol, maltitol or mannitol. One or more of such sweeteners may be present in an amount of from 0.005% to 5% by weight, for example 0.01% to 1%, for example 0.01% to 0.5%, by weight of the composition.

The dentifrice compositions of the present invention may include one or more colorants. Colorants may include pigments, dyes, lakes and agents imparting a particular color or visual quality to the composition. Any orally acceptable colorant can be used. One or more colorants may optionally be present in the compositions in an amount of from 0.001% to 2%, for example from 0.001% to 0.01%, for example from 0.001% to 0.005% of the composition by weight.

The dentifrice compositions of the present invention may include one or more humectants. Humectants can reduce evaporation and also contribute towards preservation by lowering water activity, and can also impart desirable sweetness or flavor to compositions. Suitable humectants include edible polyhydric alcohols such as glycerin, sorbitol, xylitol, propylene glycol as well as other polyols and mixtures of these humectants. Other useful materials may also include orally acceptable alcohols, or polymers, e.g., such as polyvinylmethyl ether maleic acid copolymers, polysaccharides (e.g. cellulose derivatives, for example carboxymethyl cellulose, or polysaccharide gums, for example xanthan gum or carrageenan gum). In some embodiments, the humectant can be present in an amount of from 20% to 60%, for example from 30% to 50%, for example from 40% to 45%, by weight of the composition.

The dentifrice compositions of the present invention may include a preservative. Suitable preservatives include, for example, sodium benzoate, potassium sorbate, methylisothiazolinone, paraben preservatives, for example methyl p-hydroxybenzoate, propyl p-hydroxybenzoate, and mixtures thereof.

The dentifrice compositions of the present invention may include a flavoring agent. Suitable flavoring agents include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. The flavoring agent is typically incorporated in the oral composition at a concentration of 0.01 to 3% by weight.

The dentifrice compositions of the present invention may further comprise a pH adjuster. For example, the compositions may comprise an acid or base in an amount sufficient to adjust the pH of the compositions. The desired pH of the composition of the present disclosure is from pH 7.5 to pH 10, preferably from pH 7.5 to pH 8.5, e.g., pH 7.6 to pH 8.4, pH 7.7 to pH 8.3, pH 7.8 to pH 8.1, or, e.g., about pH 8.0.

Water is present in the compositions of the present invention. Water employed in the preparation of commercial oral compositions should be deionized and free of organic impurities. Water commonly makes up the balance of the compositions and is present in an amount of from 1% to 20%, e.g., from 5% to 15%, 5% to 10%, from 7% to 15%, or from 11% to 13% by weight of the compositions. This amount of water includes the free water which is added plus that amount which is introduced with other materials or any components of the compositions described herein.

The compositions of the present invention can be manufactured following standard toothpaste formulation procedure. For example, the compositions can be manufactured as follows. Polymer gums are dispersed in glycerin with gentle stirring to make completely homogeneous gel phase. A premix is prepared by dissolving stannous fluoride and sodium saccharin in formula amounts of water. The premix solution is added to the gel phase and mixed for 12-15 minutes. Sodium bicarbonate, silica, zinc oxide and titanium dioxide are added to the mixture and mixed at low speed for 3-5 minutes for proper mixing. The mixture is then mixed at an increased speed under vacuum for 25-30 minutes to create a smooth dentifrice. Surfactants and flavoring agents are added to the dentifrice and mixed at full speed under vacuum for 12-15 minutes until homogeneous.

The following examples are further illustrative of the preferred embodiments, but it is understood that the invention is not limited thereto.

### EXAMPLES

### Example 1

Dentifrice compositions are prepared having the formulations as indicated in Table 1.

**Table 1**

| ingredient | I (wt%) | II (wt%) |
|---|---|---|
| humectant | 11.99 | 11.68 |
| demineralized water | 12.36 | 11.52 |
| thickener | 0.25 | 0.2 |
| sodium bicarbonate | 67 | 67 |
| stannous fluoride | 0.45 | 0.45 |
| synthetic amorphous silica | 3.5 | 3.5 |
| surfactant | 2.5 | 2.5 |
| flavor, sweetener and colors | 1.95 | 1.95 |
| zinc oxide | 0 | 1.2 |
| Total | 100 | 100 |

Composition I contains 67% sodium bicarbonate and 0.45% stannous fluoride. Composition II further contains 1.2% zinc oxide. The dentifrice compositions were subjected to an aging study at 40°C to determine the stability of fluoride, zinc and stannous ions in the compositions. The amounts of soluble fluoride, soluble zinc, and soluble Tin and the amount of tin in the stannous form (tin (II) were measured at initial, 1 and 3 months by standard methods. The following method was used to determine the concentration of stannous in a dentifrice composition: 5.00 grams of dentifrice is homogenously dispersed in 10.0mL of 2M citric acid in deionized water. The dispersion is treated with an excess of an exact quantity of iodine to fully consume the stannous. The mixture is titrated with 0.1N sodium thiosulfate solution using colorimetric endpoint determination with starch indicator. The titration endpoint is used to determine the quantity of stannous ions in the dentifrice. The results are shown in Table 2.

**Table 2**

| | Time | Soluble Fluoride | Soluble Zn | Zinc | Soluble Tin | Tin | Tin (ll) (titration) | % Sn |
|---|---|---|---|---|---|---|---|---|
| | | (ppm) | % | % | % | % | ppm | in Sn (II) form |
| Composition I | Initial | 1140 | | | 0.06 | 0.41 | 3266 | 96% |
| | 1M 40°C | 1122 | NA | NA | 0.03 | | 2930 | 86% |
| | 3M 40°C | 1172 | | | 0.04 | | 1133 | 33% |
| Composition II | Initial | 1157 | 0.02 | 0.96 | 0.04 | 0.38 | 2719 | 80% |
| | 1M 40°C | 1091 | 0.03 | | Not detected | | 1982 | 58% |
| | 3M 40°C | 1158 | 0.06 | | 0.03 | | 2058 | 60% |

The level of soluble metal (both zinc and tin) is low in Compositions I and II. However, the titration result shows that the proportion of tin in the stannous form in Compositions I and II is higher, compared to the proportion of tin in the stannous form in commercial stannous fluoride containing toothpastes as shown in Table 3.

**Table 3**

| Sample | Sn(II) (ppm) (Titration) | Total Sn added (ppm) | % Sn in Sn (II) form |
|---|---|---|---|
| Commercial Product A | 2379 | 6400 | 37% |
| Commercial Product B | 2020 | 6400 | 32% |
| Commercial Product C | 1828 | 6400 | 29% |
| Commercial Product D | 1613 | 6400 | 25% |
| Commercial Product E | 1721 | 6400 | 27% |
| Commercial Product F | 2672 | 6400 | 42% |
| Commercial Product G | 1689 | 3400 | 50% |
| Commercial Product H | 1086 | 6400 | 17% |
| Commercial Product I | 1093 | 3400 | 32% |
| Commercial Product J | 3389 | 6400 | 53% |

96% and 80% tin exist in the stannous form at the initial time point in Compositions I and II, respectively, whereas the proportion of tin in the stannous form (tin (II) in tested commercial stannous fluoride containing toothpastes ranges from 17% to 50%. The high proportion of tin in the stannous form in Compositions I and II might be due to the formation of insoluble stannous bicarbonate and/or stannous hydroxide due to the presence of sodium bicarbonate and the resulting high pH of the formulation. The results in Table 2 further show that the addition of zinc oxide into the sodium bicarbonate toothpaste containing stannous fluoride improves the stability of stannous ion in the formulation. After three months at 40 °C, the proportion of tin in the stannous form Compositions II remains high, compared to Composition I (60% vs. 33%). Insoluble zinc oxide may act as a way of stabilizing stannous ion via adsorption of stannous ion on the surface of zinc oxide particle.

### Example 2

Oral biofilm inhibiting efficacy of Composition II and three comparative compositions (CI, CII and CIII) was measured by in-vitro biofilm assay. The formulations of comparative compositions CI, CII and CIII are shown in Tables 4-6. Composition CIII has been clinically shown to be effective and is thus used as a positive control in the experiment.

**Table 4**

| ingredient | CI (wt%) |
|---|---|
| humectant | 48.6 |
| thickener | 0.5 |
| sodium fluoride | 0.22 |
| synthetic thickening silica | 8 |
| synthetic abrasive silica | 8 |
| surfactant | 3.47 |
| flavor, sweetener and colors | 1.68 |
| film | 0.2 |
| water | 29.33 |
| Total | 100 |

**Table 5**

| ingredient | CII (wt%) |
|---|---|
| humectant | 12 |
| demineralized water | 15.88 |
| thickener | 0.5 |
| sodium bicarbonate | 67 |
| sodium fluoride | 0.32 |
| surfactant | 2.5 |
| flavor, sweetener and colors | 1.8 |
| zinc oxide | 0 |
| Total | 100 |

**Table 6**

| ingredient | CIII (wt%) |
|---|---|
| humectant | 35.65 |
| thickener | 2.1 |
| stannous fluoride | 0.45 |
| tetrasodium pyrophosphate | 2 |
| abrasive silica | 20 |
| thickening silica | 1.5 |
| surfactant | 2.75 |
| zinc phosphate | 1 |
| flavor, sweetener and colors | 2.29 |
| trisodium citrate dihydrate | 1 |
| citric acid-anhydrous | 0.2 |
| water | 31.06 |
| Total | 100 |

Oral biofilm inhibiting efficacy of dentifrice formulations were measured by in-vitro assay using University of Manchester Growth Inhibition Model. The anaerobic model is used to provide a more sensitive indication of potential efficacy of the formula. In this experiment, saliva collected from 4 healthy volunteers and pooled together is used as inoculum. Each sample is treated in triplicate twice a day for 8 days. The biofilm is recovered after 16 treatments to measure ATP (RLU) as end point for viable bacteria. The results are shown in Table 7.

**Table 7**

| Treatment | N | Mean (Viable Bacteria as ATP RLU) |
|---|---|---|
| Composition CI (Negative control) | 9 | 4.78099 |
| Composition CII | 9 | 4.44806 |
| Composition II | 9 | 4.07889 |
| Composition CIII (Positive control) | 9 | 4.03618 |

The results show that Composition CII containing 67% sodium bicarbonate has a higher biofilm inhibiting activity, compared to Composition CI. This is expected because a high level of sodium bicarbonate is known to reduce plaque biofilms. The results further show that the biofilm inhibiting activity of Composition II is higher than that of Composition CII. This shows that despite low solubility of tin and zinc in sodium bicarbonate toothpaste formulations, these metals are both available and bioactive. The biofilm inhibiting activity of Composition II is equivalent to the biofilm inhibiting activity of the clinically tested positive control (CIII).

## Claims

1. A dentifrice composition comprising sodium bicarbonate and a stannous ion source, wherein sodium bicarbonate is present in an amount of greater than 50% by weight of the composition.

2. The composition of claim 1, wherein sodium bicarbonate is present in an amount of from 60% to 80% by weight of the composition.

3. The composition of claim 1 or 2, wherein the stannous ion source is present in an amount of from 0.01 % to 10% by weight of the composition.

4. The composition of any preceding claim, wherein the stannous ion source is stannous fluoride.

5. The composition of claim 4, wherein the composition further comprises other stannous ion source which is not stannous fluoride.

6. The composition of any preceding claim, wherein the composition comprises zinc oxide.

7. The composition of claim 6, wherein zinc oxide is present in an amount of 0.5% to 1.5% by weight of the composition.

8. The composition of any preceding claim, wherein the composition comprises zinc oxide and zinc citrate.

9. The composition of any preceding claim, wherein the composition comprises ingredients selected from one or more of buffering agents, humectants, surfactants, gum strips or fragments, breath fresheners, flavoring, fragrance, coloring, antibacterial agents, whitening agents, agents that interfere with or prevents bacterial attachment, calcium sources, and potassium salts.

10. The composition of any preceding claim, wherein the dentifrice composition does not contain any chelator that chelates stannous ions.

11. The composition of any preceding claim, wherein sodium bicarbonate is present in an amount of from 65% to 80% by weight of the composition.

12. The composition of any preceding claim, wherein sodium bicarbonate is present in an amount of from 65% to 70% by weight of the composition.

13. The composition of any preceding claim for use in a method of (i) reducing or inhibiting formation of dental caries, (ii) reducing, repairing or inhibiting pre-carious lesions of the enamel, (iii) reducing or inhibiting demineralization and promoting remineralization of the teeth, (iv) reducing hypersensitivity of the teeth, (v) reducing or inhibiting gingivitis, (vi) promoting healing of sores or cuts in the oral cavity, (vii) reducing levels of acid producing bacteria, (viii) reducing or inhibiting microbial biofilm formation in the oral cavity, (ix) reducing or inhibiting plaque formation in the oral cavity, (x) promoting systemic health, or (xi) cleaning teeth and oral cavity.

## Patentansprüche

1. Zahnreinigungsmittelzusammensetzung umfassend Natriumbicarbonat und eine Zinnionenquelle, wobei Natriumbicarbonat in einer Menge von mehr als 50 %, bezogen auf das Gewicht der Zusammensetzung, vorhanden ist.

2. Zusammensetzung nach Anspruch 1, wobei Natriumbicarbonat in einer Menge von 60 % bis 80 %, bezogen auf das Gewicht der Zusammensetzung, vorhanden ist.

3. Zusammensetzung nach Anspruch 1 oder 2, wobei die Zinnionenquelle in einer Menge von 0,01 % bis 10 %, bezogen auf das Gewicht der Zusammensetzung, vorhanden ist.

4. Zusammensetzung nach einem beliebigen vorhergehenden Anspruch, wobei die Zinnionenquelle Zinnfluorid ist.

5. Zusammensetzung nach Anspruch 4, wobei die Zusammensetzung weiterhin eine andere Zinnionenquelle umfasst, die nicht Zinnfluorid ist.

6. Zusammensetzung nach einem beliebigen vorhergehenden Anspruch, wobei die Zusammensetzung Zinkoxid umfasst.

7. Zusammensetzung nach Anspruch 6, wobei Zinkoxid in einer Menge von 0,5 % bis 1,5 %, bezogen auf das Gewicht der Zusammensetzung, vorhanden ist.

8. Zusammensetzung nach einem beliebigen vorhergehenden Anspruch, wobei die Zusammensetzung Zinkoxid und Zinkcitrat umfasst.

9. Zusammensetzung nach einem beliebigen vorhergehenden Anspruch, wobei die Zusammensetzung Bestandteile umfasst, die ausgewählt sind aus einem oder mehreren von Puffermitteln, Feuchthaltemitteln, Tensiden, Gummistreifen oder - fragmenten, Atemerfrischern, Geschmacksstoffen, Duftstoffen, Farbstoffen, antibakteriellen Mitteln, aufhellenden Mitteln, Mitteln, die die Anhaftung von Bakterien stören oder verhindern, Calciumquellen und Kaliumsalzen.

10. Zusammensetzung nach einem beliebigen vorhergehenden Anspruch, wobei die Zahnreinigungsmittelzusammensetzung keinen Chelatbildner enthält, der Zinnionen chelatiert.

11. Zusammensetzung nach einem beliebigen vorhergehenden Anspruch, wobei Natriumbicarbonat in einer Menge von 65 % bis 80 %, bezogen auf das Gewicht der Zusammensetzung, vorhanden ist.

12. Zusammensetzung nach einem beliebigen vorhergehenden Anspruch, wobei Natriumbicarbonat in einer Menge von 65 % bis 70 %, bezogen auf das Gewicht der Zusammensetzung, vorhanden ist.

13. Zusammensetzung nach einem beliebigen vorhergehenden Anspruch zur Verwendung in einem Verfahren zur (i) Verringerung oder Verhinderung der Bildung von Zahnkaries, (ii) Verringerung, Reparatur oder Verhinderung von präkariösen Läsionen des Zahnschmelzes, (iii) Verringerung oder Verhinderung der Demineralisierung und Förderung der Remineralisierung der Zähne, (iv) Verringerung der Überempfindlichkeit der Zähne, (v) Verringerung oder Hemmung von Zahnfleischentzündungen, (vi) Förderung der Heilung von Wunden oder Schnitten in der Mundhöhle, (vii) Verringerung der Menge säurebildender Bakterien, (viii) Verringerung oder Hemmung der mikrobiellen Biofilmbildung in der Mundhöhle, (ix) Verringerung oder Hemmung der Plaquebildung in der Mundhöhle, (x) Förderung der systemischen Gesundheit oder (xi) Reinigung der Zähne und der Mundhöhle.

## Revendications

1. Composition de dentifrice comprenant du bicarbonate de sodium et une source d'ions stanneux, dans laquelle le bicarbonate de sodium est présent en une quantité supérieure à 50 % en poids de la composition.

2. Composition selon la revendication 1, dans laquelle le bicarbonate de sodium est présent en une quantité de 60 % à 80 % en poids de la composition.

3. Composition selon la revendication 1 ou 2, dans laquelle la source d'ions stanneux est présente en une quantité de 0,01 % à 10 % en poids de la composition.

4. Composition selon l'une quelconque des revendications précédentes, dans laquelle la source d'ions stanneux est le fluorure stanneux.

5. Composition selon la revendication 4, dans laquelle la composition comprend, en outre, une autre source d'ions stanneux qui n'est pas le fluorure stanneux.

6. Composition selon l'une quelconque des revendications précédentes, dans laquelle la composition comprend de l'oxyde de zinc.

7. Composition selon la revendication 6, dans laquelle l'oxyde de zinc est présent en une quantité de 0,5 % à 1,5 % en poids de la composition.

8. Composition selon l'une quelconque des revendications précédentes, dans laquelle la composition comprend de l'oxyde de zinc et du citrate de zinc.

9. Composition selon l'une quelconque des revendications précédentes, dans laquelle la composition comprend des ingrédients choisis parmi un ou plusieurs éléments parmi les agents tampons, les humectants, les tensioactifs, les bandes ou fragments de gomme, les rafraîchisseurs d'haleine, les arômes, les parfums, les colorants, les agents antibactériens, les agents blanchissants, les agents qui interfèrent avec ou empêchent la fixation bactérienne, les sources de calcium et les sels de potassium.

10. Composition selon l'une quelconque des revendications précédentes, dans laquelle la composition de dentifrice ne contient aucun agent chélatant qui chélate les ions stanneux.

11. Composition selon l'une quelconque des revendications précédentes, dans laquelle le bicarbonate de sodium est présent en une quantité de 65 % à 80 % en poids de la composition.

12. Composition selon l'une quelconque des revendications précédentes, dans laquelle le bicarbonate de sodium est présent en une quantité de 65 % à 70 % en poids de la composition.

13. Composition selon l'une quelconque des revendications précédentes pour une utilisation dans un procédé (i) de réduction ou d'inhibition de la formation de caries dentaires, (ii) de réduction, de réparation ou d'inhibition des lésions précariées de l'émail, (iii) de réduction ou d'inhibition de la déminéralisation et de promotion de la reminéralisation des dents, (iv) de réduction de l'hypersensibilité des dents, (v) de réduction ou d'inhibition de la gingivite, (vi) de promotion de la guérison des plaies ou des coupures dans la cavité buccale, (vii) de réduction des niveaux de bactéries productrices d'acide, (viii) de réduction ou d'inhibition de la formation de biofilm microbien dans la cavité buccale, (ix) de réduction ou d'inhibition de la formation de plaque dans la cavité buccale, (x) de promotion de la santé systémique, ou (xi) de nettoyage des dents et de la cavité buccale.
